# EUROPEAN PATENT SPECIFICATION

(11) **EP 2 579 838 B1**
(45) Date of publication and mention of the grant of the patent: **07.01.2015**
(21) Application number: 11720536.9
(22) Date of filing: 20.05.2011
(51) Int. Cl.: A61K 8/29, A61K 8/362, A61K 8/365, A61Q 5/06

(54) **HAIR STYLING COMPOSITION**
HAARSTYLINGZUSAMMENSETZUNG
COMPOSITION DE COIFFURE

(30) Priority: 11.06.2010 EP 10165618
(43) Date of publication of application: 17.04.2013
(73) Proprietor: Unilever PLC, a company registered in England and Wales under company no. 41424, Greater London EC4Y 0DY (GB); Unilever N.V., 3013 AL Rotterdam (NL)
(72) Inventor: PAUL, Prem, Kumar, Cheyalazhagan, Bebington, Wirral Merseyside CH63 3JW (GB); PYE, Susan, Bebington, Wirral Merseyside CH63 3JW (GB)
(74) Representative: Tansley, Sally Elizabeth
(86) International application number: PCT/EP2011/058288
(87) International publication number: WO 2011/154239

(56) References cited:
- EP-A1- 0 117 360
- EP-A1- 0 530 974
- WO-A1-96/31188
- WO-A1-2009/138288
- WO-A1-2010/121924
- WO-A2-2010/028137
- US-A1- 2002 041 856

## Description

The invention relates to a method of straightening hair.

Straight, well conditioned hair is seen by some consumers as beneficial. There remains the need for a product that can decrease the volume of hair and that leaves it feeling soft, smooth and easy to comb, this is especially the case for products for use with hair straighteners.

Compositions for setting, including strainghtening and conditioning hair comprising an organic zirconate, germanate or titanate and an organosiloxane polymer are disclosed in EP0117360 A1.

The present invention relates to method of application of a product that leaves the hair looking straight, even in high humidity conditions.

The present invention relates to a method of straightening hair comprising the steps of applying to the hair on at least two successive occasions a composition comprising a titanium complex selected from the group consisting of citrate, oxalate and tartrate or their metal alkali salts.

It is advantageous if the titanium complex is titanium citrate or its metal alkali salt, particularly advantageous is titanium citrate or sodium titanium citrate.
It is highly preferred if the titanium complex is titanium citrate, in particular titanium citrate having a mole ratio of citrate to titanium from 3:1 to 2:1. especially preferred is titanium citrate having a mole ratio citrate to titanium of 2.5:1

The level of titanium compound (complex or salt) in the total composition is preferably from 0.01 to 10 wt% of the total composition, more preferably from 0.1 to 5 wt%., most preferably from 0.5 to 3 wt%.

The formulation may include conditioning materials such as surfactants, cationic conditioners suitable for hair, quaternary silicone polymers, silicone based conditioners and their emulsions, and amino functional silicones and their emulsions. Silicone based products are particularly preferred.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the emulsified silicone itself (not the emulsion or the final hair conditioning composition) is typically at least 10,000 cst. The viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use in the invention will typically have an average silicone particle size in the composition of less than 30, preferably less than 20, more preferably less than 10 microns. Most preferably the average silicone particle size of the emulsified silicone in the composition is less than 2 microns, ideally it ranges from 0.01 to 1 micron. Silicone emulsions having an average silicone particle size of ≤ 0.15 microns are generally termed microemulsions.

Particle size may be measured by means of a laser light scattering technique, using a 2600D Particle Sizer from Malvern Instruments.

Suitable silicone emulsions for use in the invention are also commercially available in a pre-emulsified form.

Examples of suitable pre-formed emulsions include emulsions DC2-1766, DC2-1784, and microemulsions DC2-1865 and DC2-1870, all available from Dow Corning. These are all emulsions/microemulsions of dimethiconol. Cross-linked silicone gums are also available in a pre-emulsified form, which is advantageous for ease of formulation. A preferred example is the material available from Dow Corning as DC X2-1787, which is an emulsion of cross-linked dimethiconol gum. A further preferred example is the material available from Dow Corning as DC X2-1391, which is a microemulsion of cross-linked dimethiconol gum.

A further preferred class of silicones for inclusion in the invention are amino functional silicones. By "amino functional silicone" is meant a silicone containing at least one primary, secondary or tertiary amine group, or a quaternary ammonium group.

Examples of suitable amino functional silicones include:
(i) polysiloxanes having the CTFA designation "amodimethicone", and the general formula:

   Ho-[Si(CH₃)₂-o-]ₓ-[Si(OH)(CH₂CH₂CH₂-NH-CH₂CH₂NH₂)-O-]_{y}-H

   in which x and y are numbers depending on the molecular weight of the polymer, generally such that the molecular weight is between about 5,000 and 500,000.
(ii) polysiloxanes having the general formula:

   RₐG₃₋ₐ-Si(OSiG₂)ₙ-(OSiG_{b}R'_{2-b})ₘ-O-SiG₃₋ₐ-R'ₐ

   in which:
   G is selected from H, phenyl, OH or C₁₋₈ alkyl, e.g. methyl;
   a is 0 or an integer from 1 to 3, preferably 0;
   b is 0 or 1, preferably 1;
   m and n are numbers such that (m + n) can range from 1 to 2000, preferably from 50 to 150;
   m is a number from 1 to 2000, preferably from 1 to 10;
   n is a number from 0 to 1999, preferably from 49 to 149, and
R is a monovalent radical of formula -C_{q}H_{2q}L in which q is a number from 2 to 8 and L is an aminofunctional group selected from the following:
- NR"-CH₂-CH₂-N(R")₂
- N(R")₂
- N⁺(R")₃A⁻
- N⁺H(R")₂A⁻
- N⁺H₂(R")A⁻
- N(R")-CH₂-CH₂-N⁺H₂(R")A⁻
in which R" is selected from H, phenyl, benzyl, or a saturated monovalent hydrocarbon radical, e.g. C₁₋₂₀ alkyl, and;
A is a halide ion, e.g. chloride or bromide.

Suitable amino functional silicones corresponding to the above formula include those polysiloxanes termed "trimethylsilylamodimethicone" as depicted below, and which are sufficiently water insoluble so as to be useful in compositions of the invention:

Si(CH₃)₃-O-[Si(CH₃)₂-O-]ₓ-[Si (CH₃)(R - NH - CH₂CH₂NH₂)-O-]y - Si (CH₃)₃

wherein x + y is a number from about 50 to about 500, and wherein R is an alkylene group having from 2 to 5 carbon atoms. Preferably, the number x + y is in the range of from about 100 to about 300.
(iii) quaternary silicone polymers having the general formula:

   {(R¹)(R²)(R³)N⁺CH₂CH(OH)CH₂O(CH₂)₃[Si(R⁴)(R⁵)-O-]ₙ-Si(R⁶)(R⁷)-(CH₂)₃-O-CH₂CH(OH)CH₂N⁺(R⁸)(R⁹)(R¹⁰)} (X⁻)₂
wherein R¹ and R¹⁰ may be the same or different and may be independently selected from H, saturated or unsaturated long or short chain alk(en)yl, branched chain alk(en)yl and C₅-C₈ cyclic ring systems;
R² thru' R⁹ may be the same or different and may be independently selected from H, straight or branched chain lower alk(en)yl, and C₅-C₈ cyclic ring systems; n is a number within the range of about 60 to about 120, preferably about 80, and
X- is preferably acetate, but may instead be for example halide, organic carboxylate, organic sulphonate or the like.

Suitable quaternary silicone polymers of this class are described in EP-A-0 530 974.

Amino functional silicones suitable for use in the invention will typically have a mole % amine functionality in the range of from about 0.1 to about 8.0 mole %, preferably from about 0.1 to about 5.0 mole %, most preferably from about 0.1 to about 2.0 mole %. In general the amine concentration should not exceed about 8.0 mole % since we have found that too high an amine concentration can be detrimental to total silicone deposition and therefore conditioning performance. The viscosity of the amino functional silicone can suitably range from about 100 to about 500,000 cst.

Specific examples of amino functional silicones suitable for use in the invention are the aminosilicone oils DC2-8220, DC2-8166, DC2-8466, and DC2-8950-114, DC7134 (all ex Dow Corning), and GE 1149-75, (ex General Electric Silicones).

Also suitable are emulsions of amino functional silicone oils with non ionic and/or cationic surfactant.

Suitably such pre-formed emulsions will have an average amino functional silicone particle size in the shampoo composition of less than 30, preferably less than 20, more preferably less than 10 microns. Most preferably the average amino functional silicone particle size in the composition is less than 2 microns, ideally it ranges from 0.01 to 1 micron. Silicone emulsions having an average silicone particle size of ≤ 0.15 microns are generally termed microemulsions.

Pre-formed emulsions of amino functional silicone are also available from suppliers of silicone oils such as Dow Corning and General Electric. Specific examples include DC929 Cationic Emulsion, DC939 Cationic Emulsion, and the non-ionic emulsions DC2-7224, DC2-8467, DC2-8177 and DC2-8154 (all ex Dow Corning).

The total amount of silicone incorporated into compositions of the invention depends on the level of conditioning desired and the material used. A preferred amount is from 0.01 to about 10% by weight of the total composition.

We have found that a total amount of silicone of from 0.3 to 5%, preferably 1 to 8%, by weight of the total composition is a suitable level.

In some aspects of this invention it is desirable if the composition comprises a styling aid.

Particularly useful as styling aids with this invention are hair styling polymers. Hair styling polymers are well known and many such polymers are available commercially which contain moieties which render the polymers cationic, anionic, amphoteric or nonionic in nature. The polymers may be synthetic or naturally derived.

Compositions for use with the method of the present invention are preferably formulated into hair care compositions with hair styling and/or hair straightening claims. The compositions are preferably used to non-permanently style human hair and, more preferably, they are packaged and labeled as such. The term non-permanently means that during the styling process the inter cystine-disulphide bonds are not broken. This means that preferably there are no reductive agents in the hair care composition. It is preferable if the composition is free of thioglycolic acid and thiolactic acid.

It is also preferable if the composition is free of metal complexes of porphyrines, naphtalocyanines, phtalocyanines, cyanobalamines and derivatives thereof.

It is preferred if the products are left on hair after application and not immediately washed off (within 10 minutes of application). Such products formulated not be immediately rinsed off are commonly known as leave on formulations.
It is particularly preferred if the compositions are applied to the hair and then not rinsed off for at least an hour. Preferred leave on formulations are mousses, gels, waxes, sprays and aerosols. Non-aerosol leave on product forms are especially preferred, in particular creams.

Hair styling waxes, creams or gels also typically contain a structurant or thickener, typically in an amount of from 0.01 % to 10% by weight of the total composition, more preferably from 0.1 to 5 wt%.

Examples of suitable structurants or thickeners are polymeric thickeners such as carboxyvinyl polymers. A carboxyvinyl polymer is an interpolymer of a monomeric mixture comprising a monomeric olefinically unsaturated carboxylic acid, and from about 0.01 % to about 10% by weight of the total monomers of a polyether of a polyhydric alcohol. Carboxyvinyl polymers are substantially insoluble in liquid, volatile organic hydrocarbons and are dimensionally stable on exposure to air. Suitably the molecular weight of the carboxyvinyl polymer is at least 750,000, preferably at least 1,250,000, most preferably at least 3,000,000. Preferred carboxyvinyl polymers are copolymers of acrylic acid cross-linked with allylsucrose or allylpentaerythritol as described in US Patent 2,798,053. These polymers are provided by B.F.Goodrich Company as, for example, CARBOPOL 934, 940, 941 and 980. Other materials that can also be used as structurants or thickeners include those that can impart a gel-like viscosity to the composition, such as water soluble or colloidally water soluble polymers like cellulose ethers (e.g. methylcellulose, hydroxyethylcellulose, hydroxypropylmethylcellulose and carboxymethylcellulose), guar gum, sodium alginate, gum arabic, xanthan gum, polyvinyl alcohol, polyvinyl pyrrolidone,hydroxypropyl guar gum, starch and starch derivatives, and other thickeners, viscosity modifiers, gelling agents, etc. Particularly preferred thickeners are those based on acrylate, such as sodium acrylate copolymer, a commercial example of this polymer is Tinovis CD Ex Ciba. It is also possible to use inorganic thickeners such as bentonite or laponite clays. Such styling products frequently include a carrier and further additional components. The carriers and additional components required to formulate such products vary with product type and can be routinely chosen by one skilled in the art. The following is a description of some of these carriers and additional components.

The pH of the formulation is preferably from 2.5 to 8 at 25°C. More preferably from 3 to 6.

Hair care compositions of the present invention can comprise a carrier, or a mixture of such carriers, which are suitable for application to the hair. The carriers are present at from about 0.5% to about 99.5%, preferably from about 5.0% to about 99.5%, more preferably from about 10.0% to about 98.0%, of the composition. As used herein, the phrase "suitable for application to hair" means that the carrier does not damage or negatively affect the aesthetics of hair or cause irritation to the underlying skin.

Compositions according to the invention comprise a buffer or pH adjuster. Preferred buffers or pH adjusters include weak acids and bases such glycine/sodium hydroxide, citric acid, lactic acid, succinic acid, acetic salt and salts thereof. Frequently a mixture of buffering system is used such as sodium citrate and citric acid.

Carriers suitable for use with hair care compositions of the present invention include, for example, those used in the formulation of hair sprays, mousses, tonics, waters, creams gels, shampoos, conditioners, and rinses. The choice of appropriate carrier will depend on the particular product to be formulated. The carriers used herein can include a wide range of components conventionally used in hair care compositions. The carriers can contain a solvent to dissolve or disperse the styling compound being used, with water, the C₁-C₆ alcohols, lower alkyl acetate and mixtures thereof being preferred. The carriers can also contain a wide variety of additional materials such as acetone, hydrocarbons (such as isobutane, hexane, decene), halogenated hydrocarbons (such as Freons) and volatile silicones such as cyclomethicone.

When the hair care composition is a hair spray, tonic, gel, or mousse the preferred solvents include water, ethanol, volatile silicone derivatives, and mixtures thereof. The solvents used in such mixtures may be miscible or immiscible with each other. Mousses and aerosol hair sprays can also utilise any of the conventional propellants to deliver the material as a foam (in the case of a mousse) or as a fine, uniform spray (in the case of an aerosol hair spray). Further general ingredients suitable for all product forms include, sun-screening agents, anti-dandruff actives, carboxylic acid polymer thickeners for hair shampoo and conditioner compositions and emulsifiers for emulsifying the various carrier components of the compositions of the invention.

The compositions of the present invention may also contain adjuncts suitable for hair care. Generally such ingredients are included individually at a level of up to 2, preferably up to 1 wt% of the total composition. Suitable hair care adjuncts, include amino acids, sugars and ceramides.

The composition preferably comprises a perfume.

Compositions of the invention may comprise a surfactant. Surfactants are particularly useful as emulsifiers and in shampoo and conditioners.

The method of the invention comprises applying compositions of the invention preferably followed by a heating step to a temperature above 50°C, more preferably above 100°C, more preferably above 180°C.

The following non-limiting Examples further illustrate the preferred embodiments of the invention. All percentages referred to in the examples and throughout this specification are by weight based on total weight unless otherwise indicated.

### Examples

### Experiment 1

8 dark brown EU wavy # 6 switches (25cm in length and 2gm in weight) were washed with a base wash comprising 14% SLES , rinsed and treated with a product described below. After treatment the switches were ironed and left in a humidity chamber at 30°C and 80%RH for one hour. The procedure was repeated for 5 successive days with one cycle per day.

**Table 1.**

| Treatment | **Treatment with** | | | |
|---|---|---|---|---|
| | switches 1 & 2 | switches 3 & 4 | switches 5 & 6 | switches 7 & 8 |
| treatment cycle 1 | water | 2% Ti citrate* | 2% Ti citrate* | 2% Ti citrate* |
| treatment cycle 2 | water | water | 2% Ti citrate | 2% Ti citrate |
| treatment cycle 3 | water | water | 2% Ti citrate | 2% Ti citrate |
| treatment cycle 4 | water | water | water | 2% Ti citrate |
| treatment cycle 5 | water | water | water | 2% Ti citrate |

| | | | | |
|---|---|---|---|---|
| * titanium citrate , the citrate to titanium mole ratio being 2.5 to 1 | | | | |

The switches were image analysed and the length, volume etc. of the switches were obtained. The results are shown in tables 2 and 3. These tables give the average volumes and the corresponding standard errors.

**Table 2**

| | **average volumes** | | | |
|---|---|---|---|---|
| | switches 1 & 2 | switches 3 & 4 | switches 5 & 6 | switches 7 & 8 |
| treatment cycle 1 | 11895.6 | 12159.1 | 10601.8 | 10890.5 |
| treatment cycle 2 | 13522.1 | 12515.0 | 11605.4 | 11808.9 |
| treatment cycle 3 | 12714.9 | 11853.0 | 4643.8 | 6023.4 |
| treatment cycle 4 | 12468.1 | 11831.8 | 9926.3 | 5961.2 |
| treatment cycle 5 | 11927.7 | 11152.4 | 11306.3 | 6983.3 |

**Table 3**

| | **std error to average volumes** | | | |
|---|---|---|---|---|
| | switches 1 & 2 | switches 3 & 4 | switches 5 & 6 | switches 7 & 8 |
| treatment cycle 1 | 159.78 | 450.84 | 1849.27 | 2243.27 |
| treatment cycle 2 | 156.67 | 1140.07 | 510.00 | 867.51 |
| treatment cycle 3 | 877.17 | 575.01 | 97.01 | 951.86 |
| treatment cycle 4 | 579.49 | 854.38 | 801.86 | 1506.81 |
| treatment cycle 5 | 304.83 | 416.79 | 774.47 | 766.66 |

All "volume" measurements are in mm^2 and are actually 2-d projected areas of the switch images.

From the above tables, switches treated with Ti citrate all 5 days show progressive benefits i.e. the benefits increase with treatment cycles as shown in the table below

**Table 4**

| | **average volume after high humidity** | |
|---|---|---|
| | 1 st 2 treatments | 3rd - 5th treatments |
| Ti citrate treated | 11349.68 | 6322.62 |
| std. error | 1017.07 | 542.89 |
| | | |
| water treated | 12708.86 | 12370.24 |
| std. error | 478.32 | 318.58 |
| | | |
| % benefit increase | 10.7 | 48.9 |
| | | |
| Significance | p = 87% | p > 99% |

From the table it can also be seen that there is almost a 50% increase in benefits with successive use of Ti citrate.

Table 5 exemplifies a combing cream suitable for successive use.

**Table 5 -Combing Cream**

| **Trade Name** | **Chemical (INCI) Name** | **Supplier** | **Example A % w/w** | **Example 1 % W/W** |
|---|---|---|---|---|
| Brij 72 | Steareth-2 | Uniqema | 0.18 | 0.18 |
| Myrj 52S | PEG-40 Steareth-2 | Uniqema | 0.45 | 0.45 |
| Hydrenol MY | Cetearyl Alcohol | Cognis | 1.50 | 1.50 |
| Tinovis CD | Sodium Acrylates Copolymer, Mineral oil, PPG-1 Trideceth-6 | Ciba | 0.80 | 0.80 |
| DC 7134 | Dimethyl methylaminoethyllamine isobutyl siloxane | Dow Corning | 0.740 | 0.740 |
| DC 1788 | Dimethiconol/Dimethiconol/ Silsesquioxane Copolymer | Dow Corning | 0.800 | 0.800 |
| Estol 1517 | Isopropyl Palmitate | Croda | 1.00 | 1.00 |
| Glycerine | Glycerine | BDH | 2.00 | 2.00 |
| | Sodium titanium citrate | | 0 | 2.00 |
| | Water and minors | | To 100% | To 100% |

## Claims

1. A method of straightening hair comprising the steps of applying to the hair on at least two successive occasions a composition comprising a titanium complex selected from the group consisting of citrate, oxalate and tartrate or the metal alkali salt of these.

2. A method according to claim 1 in which the composition according to any preceding claim comprises titanium citrate or sodium titanium citrate.

3. A method according to claim 1 or claim 2 in which the titanium complex is titanium citrate having a mole ratio of citrate to titanium from 3:1 to 2:1.

4. A method according to any preceding claim in which the level of titanium complex or salt thereof is from 0.01 to 10 wt%.

5. A method according to any preceding claim in which the composition further comprises a silicone.

6. A method according to any preceding claim in which the composition is applied and is not followed within 1 hour by a rinsing step.

## Patentansprüche

1. Verfahren zum Glätten des Haars,
das die Schritte des Auftragens einer Zusammensetzung in mindestens zwei aufeinanderfolgenden Vorgängen auf das Haar aufweist, wobei die Zusammensetzung einen Titankomplex aufweist, der aus der Gruppe ausgewählt ist, die aus Citrat, Oxalat und Tartrat oder einem Alkalimetallsalz davon besteht.

2. Verfahren nach Anspruch 1,
wobei die Zusammensetzung gemäß dem vorstehenden Anspruch Titancitrat oder Natriumtitancitrat aufweist.

3. Verfahren nach Anspruch 1 oder 2,
wobei der Titankomplex Titancitrat mit einem Molverhältnis zwischen Citrat und Titan von 3:1 bis 2:1 ist.

4. Verfahren nach einem der vorstehenden Ansprüche,
wobei die Menge des Titankomplexes oder eines Salzes davon 0,01 bis 10 Ges.-% beträgt.

5. Verfahren nach einem der vorstehenden Ansprüche,
wobei die Zusammensetzung ferner ein Silicon aufweist.

6. Verfahren nach einem der vorstehenden Ansprüche,
wobei die Zusammensetzung aufgebracht wird und innerhalb von 1 Stunde kein Spülschritt folgt.

## Revendications

1. Procédé de lissage des cheveux comprenant les étapes d'application aux cheveux en au moins deux occasions successives d'une composition comprenant un complexe de titane choisi dans le groupe constitué de citrate, d'oxalate et de tartrate ou du sel de métal alcalin de ceux-ci.

2. Procédé selon la revendication 1, dans lequel la composition selon l'une quelconque des revendications précédentes comprend du citrate de titane ou du citrate de sodium titane.

3. Procédé selon la revendication 1 ou la revendication 2, dans lequel le complexe de titane est le citrate de titane présentant un rapport molaire de citrate au titane de 3:1 à 2:1.

4. Procédé selon l'une quelconque des revendications précédentes, dans lequel la teneur en complexe de titane ou sel de celui-ci est de 0,01 à 10 % en poids.

5. Procédé selon l'une quelconque des revendications précédentes, dans lequel la composition comprend de plus un silicone.

6. Procédé selon l'une quelconque des revendications précédentes, dans lequel la composition est appliquée et n'est pas sur une durée de 1 heure d'une étape de rinçage.
